# EUROPEAN PATENT APPLICATION

(11) **EP 3 106 226 A1**
(43) Date of publication of application: **21.12.2016**
(21) Application number: 15172663.5
(22) Date of filing: 18.06.2015
(51) Int. Cl.: B01L 7/00, B01L 9/06, B01L 3/00, C12M 3/00, C12M 1/02

(54) **METHOD AND APPARATUS FOR MINIMIZING CHANGE IN TEMPERATURE OF FOLLICLE FLUID WHILE TRANSFER**

(71) Applicant: Shivani Scientific Industries Private Limited, 401104 Mumbai (IN)
(72) Inventor: Kale, Ravikant, 401104 Mumbai (IN); Modi, Ashish, 401104 Mumbai (IN)
(74) Representative: Nordmeyer, Philipp Werner

(57) **Abstract**

Disclosed is a method for maintaining temperature of a human follicle fluid containing a human oocyte. The method may comprise a warm dry block, wherein the warm dry block may be adapted to hold a plurality of test tubes. The warm dry block may further be adapted to constantly maintain the temperature of the human follicle fluid containing human oocyte in the tube. In one aspect, when the tube is displaced from the warm dry block, an outer jacket may facilitate to maintain the temperature until the tube is again placed in the warm dry block. In one aspect, the tube is wrapped around with the outer jacket, wherein the outer jacket may be composed of Aluminum (Al) or Aluminum (Al) alloy.

## Description

### TECHNICAL FIELD

The invention described herein, in general, relates to a method and apparatus for controlling and maintaining minimal change in temperature of a human follicle fluid in a test tube.

### BACKGROUND

Millions of couples across the world are facing challenges related to infertility. This is leading to demand for extraction and cryopreservation of human oocytes for In-vitro fertilization (IVF) procedures. The human oocyte aspirated from the female body as a part of the follicle fluid is extremely sensitive to temperature. Even while cryopreservation, the human oocyte may be extremely sensitive to cooling and temperature variations. Thus it is critical that during In-vitro fertilization (IVF) procedure, optimal physiological conditions such as temperature (within a range) is maintained for preserving the viability of an embryo. Even a small or sudden variation in the temperature may significantly compromise in growth of an embryo. In view of this, many warming devices such as incubators, warming stages, and dry block heaters have been designed and used in the IVF labs that are adapted to constantly maintain the temperature of the follicle fluid and embryo. However, for routine Assisted Reproductive Technologies (ART), the temperature fluctuations can easily occur in such warming devices whenever oocytes or embryos are transported in a test tube.

### SUMMARY

Before the methods and apparatus, are described, it is to be understood that this invention is not limited to the particular methodologies described, as there can be multiple possible embodiments which are not expressly illustrated in the present disclosure. It is also to be understood that the terminology used in the description is for the purpose of describing the particular versions or embodiments only, and is not intended to limit the scope of the present application. This summary is provided to introduce concepts related to maintaining temperature of follicle fluid, contained in a test tube, in transit and the concepts are further described below in the detailed description. This summary is not intended to identify essential features of the disclosure nor is it intended for use in determining or limiting the scope of the disclosure.

In one implementation, an apparatus and a method for maintaining temperature of follicle fluid, contained in a test tube, in transit is disclosed. In order to maintain the temperature of the follicle fluid, the apparatus may comprise a warm dry block capable of holding a plurality of test tubes. It may be understood that the warm dry block is pre-heated by a heating device to maintain the temperature of the follicle fluid extracted into the test tube. The test tube holding the extracted follicle fluid may be displaced from the warm dry block and transferred to other places including a laboratory for In-vitro fertilization (IVF) process. In such scenario, the test tube, containing the follicle fluid, may be exposed to ambient air eventually affecting the temperature and thereby affecting the viability of the oocyte present in the follicle fluid.

In order to maintain the temperature and prevent change in temperature of the follicle fluid during transition from one location to another, the warm dry block transfers heat to an outer jacket wrapped around each test tube containing the follicle fluid. The outer jacket may facilitate to maintain the temperature of the follicle fluid when the test tube along with the outer jacket is carried from one location to other. It may be understood that the outer jacket may be made up of Aluminum (Al) or Aluminum (Al) alloy which facilitates to maintain the temperature. Thus, in this manner, the outer jacket may be utilized to cover the test tube containing the follicle fluid when the follicle fluid needs to be transferred from one place to the other.

### BRIEF DESCRIPTION OF THE DRAWINGS

The foregoing detailed description of embodiments is better understood when read in conjunction with the appended drawing. For the purpose of illustrating the disclosure, there is shown in the present document example constructions of the disclosure.

The detailed description is described with reference to the accompanying figure. In the figure, the left-most digit(s) of a reference number identifies the figure in which the reference number first appears. The same numbers are used throughout the drawings to refer like features and components.

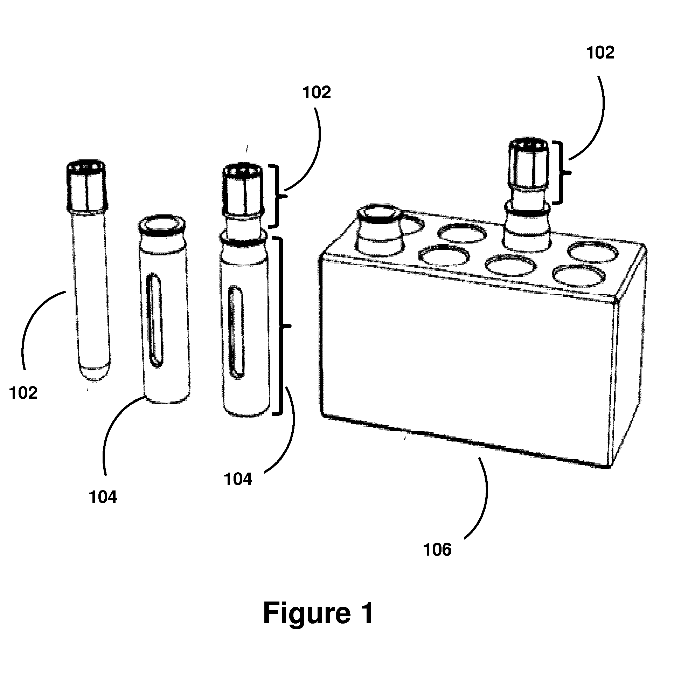

Figure 1 illustrates a warm dry block holding a plurality of test tubes is shown, in accordance with an embodiment of the present disclosure.

### DETAILED DESCRIPTION

Some embodiments of this disclosure, illustrating all its features, will now be discussed in detail. The words "comprising," "having," "containing," and "including," and other forms thereof, are intended to be equivalent in meaning and be open ended in that an item or items following any one of these words is not meant to be an exhaustive listing of such item or items, or meant to be limited to only the listed item or items. It must also be noted that, the singular forms "a," "an," and "the" include plural references unless the context clearly dictates otherwise. Although any methods similar or equivalent to those described herein can be used in the practice or testing of embodiments of the present disclosure, the exemplary methods are now described. The disclosed embodiments are merely exemplary of the disclosure, which may be embodied in various forms.

Various modifications to the embodiment will be readily apparent to those skilled in the art and the generic principles herein may be applied to other embodiments. However, one of ordinary skill in the art will readily recognize that the present disclosure is not intended to be limited to the embodiments illustrated, but is to be accorded the widest scope consistent with the principles and features described herein.

In one implementation, the present disclosure discloses a method and apparatus for maintaining temperature of a test tube having human follicle fluid further containing human oocyte for the treatment of infertility using In-Vitro Fertilization (IVF), universally known as the Test Tube Baby. As part of the IVF procedure, it is required that the human oocyte (egg) is extracted from a female patient in an operation theatre with the patient under anesthesia. In order to extract the human oocyte, a device such as vacuum generator, commonly known as 'aspiration pump' or 'aspirator' may be employed. In one aspect, the human oocyte suspended in the follicle fluid may be extracted in a test tube. The human follicle fluid extracted in the test tube may be required to be kept under constant temperature as per the specifications of In-Vitro Fertilization (IVF).

Referring to Figure 1, an apparatus comprises a warm dry block 106 for maintaining temperature of human follicle fluid, is disclosed. It may be understood that the warm dry block 106 is capable of holding a plurality of test tubes 102-1, 102-2, 102-N, collectively referred to as a test tube 102, in a plurality of place holders. In other words, each place holder in the warm dry block 106 is capable holding a test tube 102.

When the follicle fluid is first extracted from a patient's body in the Operation theatre, a person/nurse is engaged for holding the test tube 102 in which the human follicle fluid containing the human oocyte is extracted from the female patient. As the human follicle fluid is required to be maintained within a pre-defined temperature range, the test tube 102, containing the human follicle fluid, is placed in the place holder of the warm dry block 106. It may be understood that the temperature of the human follicle fluid is maintained by pre-heating the warm dry block 106. In one aspect, the warm dry block 106 is pre-heated by a heating device to maintain the temperature of the follicle fluid. In one aspect, the heating device may be coupled with the warm dry block 106. In one aspect, the warm dry block 106 may maintain the temperature within the pre-defined temperature range of 32º C to 42º C.

The warm dry block 106 may maintain the temperature only until the test tube 102, containing the human follicle fluid, has been removed from the warm dry block 106. Once the test tube 102 is displaced from the warm dry block 106 and transferred to another place, the test tube 102 containing the follicle fluid may be exposed to ambient atmosphere eventually affecting the temperature and thereby oocyte present in the follicle fluid.

The test tubes are usually made of glass body. In order to minimize the change in the temperature of the follicle fluid during transition from one place to another, the test tube 102 is provided with an outer jacket 104, capable of containing heat as shown in the figure 1. The outer jacket 104 maintains the temperature within the pre-defined temperature range. In one aspect, the outer jacket 104 may be in surface contact with the outer surface of the test tube 102 in order to regulate and maintain the temperature. It may be understood that the outer jacket 104 may be composed of Aluminum (Al) material. In one aspect, the outer jacket 104 may facilitate to maintain the temperature of the human follicle fluid containing the human oocyte during transition of the test tube 102 from one place to another. The outer jacket 104, in this manner minimizes the change in temperature of the test tube and its contents and preserves the viability of the human oocyte by keeping the temperature within the pre-defined range.

In one aspect, the outer jacket 104 itself may be heated using the warm dry block 106 in order to maintain the temperature. The temperature of outer jacket may be selectively controlled using the temperature control means provided in the dry clock 106.

In one aspect, the test tube 102 may ensure that cells or tissue are lifted into suspension at the lowest possible speeds for promoting high cell yields by preventing cell shearing.

In one embodiment the outer jacket 104 of the test tube may have a longitudinal slit so as to let the contents of the test tube be visible to an operator.

Although implementations for maintaining the temperature of the human follicle fluid containing human oocyte have been described in language specific to structural features and/or methods, it is to be understood that the implementations and/or embodiments are not necessarily limited to the specific features or methods described.

## Claims

1. An apparatus for minimizing change in temperature of a follicle fluid contained in a test tube 102, the apparatus comprising:
a warm dry block 106 capable of holding one or more test tubes, wherein the warm dry block 106 is pre-heated by a heating device, and wherein the warm dry block 106 transfers heat to an outer jacket 104 wrapped around each of the test tubes 102 containing follicle fluid, and wherein the outer jacket 104 is **characterized in** minimizing change in temperature of the follicle fluid when at least one or more test tubes 102, is pulled out of the warm dry block.

2. The apparatus of claim 1, wherein the temperature is pre-defined by an operator.

3. The apparatus of claim 1, wherein the warm dry block 106 and the outer jacket 104 is made up of aluminum (Al) or aluminum (Al) alloy.

4. The apparatus of claim 1, wherein the warm dry block 106 comprises a plurality of place holders, and wherein each place holder is capable of holding the test tube 102.

5. A method for minimizing change in temperature of a follicle fluid contained in a test tube 102, the method comprising
placing a plurality of test tubes in a warm dry block 106, wherein the warm dry block 106 is pre-heated by a heating device;
transferring heat to an outer jacket 104 wrapped around each test tube 102 containing follicle fluid; and
maintaining temperature of the follicle fluid when a test tube 102, of the plurality of test tubes, along with the outer jacket 104 is pulled out of the warm dry block.

6. The method of claim 5, wherein the temperature is pre-defined by a operator.

7. The method of claim 5, wherein the warm dry block 106 and the outer jacket 104 is made up of aluminum (Al) or aluminum (Al) alloy.

8. The method of claim 5, wherein the warm dry block 106 comprises a plurality of place holders, and wherein each place holder is capable of holding the test tube 102.

9. A test tube 102 capable of minimizing change in temperature of a follicle fluid contained in such test tube 102, the said test tube 102 **characterized in** having an outer jacket 104 wrapped around it that is capable of being heated to desired temperature using a dry block 106.

10. The test tube of claim 9, wherein outer jacket 104 is made up of aluminum (Al) or aluminum (Al) alloy.
